Europäisches Patentamt

European Patent Office

Office européen des brevets

(11) Publication number: **0 410 557 A2**

# EUROPEAN PATENT APPLICATION

(21) Application number: **90301575.8**

(22) Date of filing: **14.02.90**

(51) Int. Cl.⁵: **A61M 25/01**

(30) Priority: **26.07.89 US 386889**

(43) Date of publication of application:
**30.01.91 Bulletin 91/05**

(84) Designated Contracting States:
**BE DE FR GB LU NL**

(71) Applicant: **INTERVENTIONAL TECHNOLOGIES INC**
**4949 Viewridge Avenue**
**San Diego California 92123(US)**

(72) Inventor: **Gomringer, Gary**
**1880 Starvation Mountain Road**
**Escondido, California 92025(US)**

(74) Representative: **Coxon, Philip et al**
**Eric Potter & Clarkson St. Mary's Court St.**
**Mary's Gateate**
**Nottingham NG1 1LE(GB)**

(54) **A guide wire.**

(57) A guide wire comprises a main shaft which has a tapered distal end with an anchor formed at the tip of the distal end. A ball-shaped abutment is formed over the anchor at the distal end of the guide wire to limit movement of an over-the-wire catheter past the abutment. The portion of the main shaft immediately proximal to the abutment is flattened into a ribbon to bias bending of the guide wire's distal end and a radiopaque coil surrounds the ribbon and has one end attached to the abutment and its other end attached to the main shaft.

EP 0 410 557 A2

This invention pertains generally to guide wires for devices which are inserted over-the-wire into the veins or arteries of a patient. More particularly, this invention pertains to guide wires which control and limit the extent of insertion of an over-the-wire device into a patient. This invention is particularly, but not exclusively, useful with over-the-wire devices used in atherectomy procedures.

The use of guide wires to position catheters and medical devices within the body of a patient is well known in the medical field. Further, their efficacy has been well established for several medical procedures. Specifically, guide wires are routinely used to position catheters which are useful for both the infusion of medical solutions into the body and the drainage of fluids from the body. Also, guide wires are being increasingly used for the precise positioning of medical devices within the body. Particularly, this is so for medical devices which require insertion deep into the body of the patient.

While guide wires are seldom used for the relatively short catheters which are intended to merely gain access to an area of the body (e.g. the bladder), guide wires are almost always used in positioning the longer catheters which are designed to proceed deep into the body through a vein or an artery to the area where the medical device associated with the catheter is to be used. As can be appreciated, such placement is often extremely difficult and requires very specialized techniques.

Catheter placement devices typically fall into two categories. In the first category are guide catheters which are positioned in the body to provide a lumen through which the operative catheter is positioned. This type of placement device is most frequently used for the shorter catheters. On the other hand, longer catheters are generally over-the-wire catheters. As the name implies, these catheters are designed to slide over guide wires which are prepositioned to steer the catheter into the body toward its intended operative position. The present invention is concerned with such a guide wire.

As implied above, guide wires are extremely helpful during insertion of a catheter into the deeper recesses of the body. This is so because the catheter itself often lacks the steerability necessary for proper placement. Where extensive insertion of a catheter into a body is necessary for its proper placement, this disadvantage is profound.

Recent advancements in technology have made atherectomy devices very acceptable for procedures employed to clear blocked arteries. Importantly, unlike many of the other procedures and devices used for this purpose i.e. angioplasty, atherectomy procedures require cutting the plaque, or other obstructive material, from the inside of the artery. The excised tissue is then subsequently removed from the artery rather than being left in place. As can be easily appreciated, it is absolutely crucial during such a procedure that the cutting device be controlled and not allowed to cut through the arterial wall. The present invention recognizes that a properly designed guide wire is very useful for this purpose.

Several examples of guide wires have been previously disclosed. Typical of these examples are US Patent No. 3,731,671 and US Patent No. 3,789,841. Further, the use of a guide wire for a rotary catheter system has been disclosed in US Patent No. 4,732,154. All of these devices, however, are intended to do no more than provide means to position the operative catheter over the positioned guide wire.

The present invention recognizes that surgical procedures may sometimes be best accomplished if both the guide wire and the over-the-wire catheter can be advanced simultaneously. Further, the present invention recognizes that for certain medical devices there is a need for a device which will mechanically limit the length to which an over-the-wire catheter and its associated device can be inserted into the body. Still further, the present invention recognizes the need for a guide wire which, when withdrawn from the body, is useful for retrieving the medical device it has guided into the operational location in the body.

It is an object of this invention to provide an improved guide wire for an over-the-wire medical device.

According to one aspect of this invention there is provided a guide wire for positioning an over-the-wire medical device which guide wire comprises:
a main shaft having a proximal end and a distal end; and
an abutment on said distal end of said main shaft to prevent movement of said medical device past said abutment.

According to another aspect of this invention there is provided a guide wire for positioning an over-the-wire medical device within the artery of a human which comprises:
a main shaft having a proximal end and a distal end; and
an abutment attached to said distal end of said main shaft to prevent movement of said medical device past said abutment.

Preferably said main shaft is formed with a flat ribbon proximal said abutment to bias bending of said main shaft.

The guide wire may further comprise a helically wound wire coil which is positioned in a surrounding relationship over said ribbon. Said coil may have one end attached to said abutment and an opposite end attached to said main shaft.

The main shaft may be made of stainless steel. The coil and the abutment may be made of gold alloy. The abutment may be ball-shaped.

According to another aspect of this invention there is provided a guide wire for positioning an over-the-wire medical device which comprises: a main shaft having a proximal end and a distal end, said main shaft having a tapered region and a flat ribbon which is distal to the tapered region to bias the bending of said main shaft at said ribbon; and an abutment attached to said ribbon at said distal end of said main shaft to prevent movement of said medical device past said abutment.

Preferably the guide wire further comprises a coil having a first end and a second end and is surroundingly positioned over said ribbon with said first end attached to said abutment and said second end attached to said tapered region of said main shaft.

The guide wire may further comprise an anchor integrally attached to said ribbon for providing attachment means for said abutment.

The cross-sectional dimensions of said coil may be dimensionally less than the cross-sectional dimensions of said abutment.

The coil may be helically wound wire and may be surroundingly positioned over at least a portion of said tapered region.

The portion of said coil in surrounding relationship with said ribbon may be more openly wound than is the portion of said coil in surrounding relationship with said tapered region to provide more bending flexibility for said guide wire at said ribbon.

The abutment may be spherical-shaped, or hemispherical-shaped, or parabolic-shaped.

According to another aspect of this invention there is provided a guide wire operatively engageable with an over-the-wire catheter which comprises: a main shaft having a tapered distal end; and a sheath engageable with said distal end, said sheath formed with an abutment for preventing movement of said catheter past said abutment.

Preferably, the guide wire further comprises an anchor integrally attached to said distal end of said main shaft and the sheath may be formed to surroundingly engage said anchor.

The distal end may be tapered to create a flat ribbon to establish a biased direction for bending said distal end of said guide wire.

The sheath may be a radiopaque polymer.

The guide wire of one embodiment of the present invention comprises a main shaft that is tapered at its distal end to form a flat ribbon which establishes a biased direction for bending the distal end of the guide wire. Integrally attached to the distal end of the ribbon may be a ball-shaped abutment.

A flexible radiopaque coil may be provided which surrounds and covers the ribbon and part of the tapered portion immediately proximal to the ribbon. The coil itself may comprise a helically-shaped wire which may be integrally attached at its proximal end to the tapered region of the main shaft and may be integrally attached at its distal end to the ball-shaped abutment. Preferably, the coil is made of a gold alloy.

In an alternative embodiment, a sheath of radiographic polymeric material may be provided instead of a coil. The sheath of radiographic polymeric material may be placed to surroundingly engage the distal end of the guide wire over the ribbon region and a part of the tapered portion immediately proximal to the ribbon. In the alternative embodiment, an abutment is integrally formed as part of the sheath.

In combination with the main shaft, the abutment of the preferred embodiment or the sheath of the alternative embodiment provide a guide wire for over-the-wire catheters which prevents movement of the catheter beyond the point where the catheter makes contact with the abutment.

In light of the above, it is an advantage of the present invention that it provides a steerable guide wire which will mechanically limit the length to which a catheter can advance into the body of a patient while the catheter is surrounding the guide wire. Another advantage of the present invention is that it provides a guide wire which can be advanced into the body of a patient either independently or in combination with a over-the-wire catheter. Yet another advantage of the present invention is that it provides a guide wire which is useful for retrieving the medical device it has guided into position. Still another advantage of the present invention is that it provides a radiopaque guide wire which is easy to use. Yet another advantage of the present invention is that it provides a guide wire which is relatively easy to manufacture and is cost-effective.

Reference is now made to the accompanying drawings in which:

Figure 1 is a partial perspective view of the guide wire in cooperative engagement with an over-the-wire atherectomy device;

Figure 2 is a cross-sectional view of the guide wire as seen along the line 2-2 in Figure 1;

Figure 3 is a cross-sectional view of the guide wire as seen along the line 3-3 in Figure 1;

Figure 4 is a cross-sectional view of an alternate embodiment of a radiopaque coil which can be used with the guide wire;

Figure 5 is a partial perspective view of an alternate embodiment of the guide wire incorporating a polymer sheath; and

Figure 6 is a cross-sectional view of the alternate embodiment of the guide wire as seen along the line 6-6 in Figure 5.

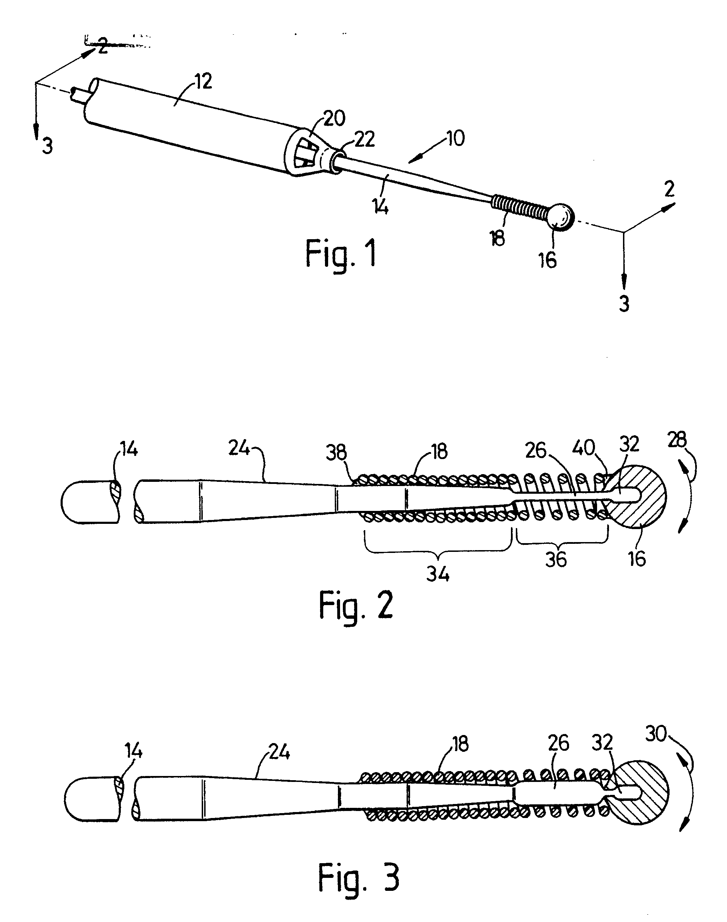

Referring initially to Figure 1, the guide wire of the present invention is generally designated 10 and shown in operative cooperation with an over-the-wire catheter 12. More specifically, in Figure 1 it can be seen that guide wire 10 comprises a main shaft 14 which has a ball-shaped abutment 16 attached at its distal end. Preferably, main shaft 14 is made of stainless steel and abutment 16 is made of a radiopaque 88/12 gold alloy, i.e. the alloy is eighty- eight percent gold and twelve percent nickel, although other alloys of gold can also be used. Abutment 16 can be attached to main shaft 14 in any manner well known in the art, such as by brazing or molding. Abutment 16 can actually be any of various shapes. For instance, abutment 16 can be either hemishperical or parabolic in its configuration. Regardless of the particular shape, the important structural considerations for abutment 16 are first, that the end be blunt in order to minimize the possibility of inadvertently puncturing an arterial wall as guide wire 10 is being positioned; and second, that abutment 16 be sufficiently large to prevent insertion of an over-the-wire catheter past abutment 16.

A radiopaque helical spring coil 18 surrounds the portion of main shaft 14 which is immediately proximal to abutment 16. Coil 18 acts as a radiopaque marker and as a smooth transitional structure between main shaft 14 and abutment 16. It is operatively attached to both abutment 16 and main shaft 14 in a manner to be subsequently disclosed.

Figure 1 also shows that catheter 12 may have a cutter 20 attached to its distal end which is preferably of the type disclosed in a co-pending application for an invention entitled "Cutter for Atherectomy Device," U.S. Application Serial No. 213,691, which was filed on June 30, 1988 and which is assigned to the same assignee as the present invention. As shown, cutter 20 is formed with a hollow cylindrical tip 22 which slidingly surrounds main shaft 14 of guide wire 10. Importantly, although tip 22 can slide along the entire length of main shaft 14, the inner diameter of tip 22 is sufficiently smaller than the outer diameter of ball-shaped abutment 16 to prevent movement of tip 22 past abutment 16.

Referring now to Figure 2, it will be seen that main shaft 14 comprises a tapered region 24 which extends from an intermediate point on main shaft 14 toward the distal end of guide wire 10. It is to be understood that the majority of main shaft 14 is proximal to region 24 and is substantially of constant diameter. Within the region 24, the taper of main shaft 14 is substantially uniform with decreasing diameter in the distal direction. Main shaft 14, as it extends distally beyond region 24, is flattened into a ribbon 26 which facilitates the bending of the distal end of guide wire 10 in the directions indicated by arrow 28. As will be appreciated by the skilled artisan, the length and cross-sectional dimensions of flattened ribbon 26 can be varied during manufacture to provide a range of flexibilities and steering characteristics. Further, it is to be understood that a bending of the distal end of guide wire 10 can establish a biased deflection which is useful for steering guide wire 10 through a preselected artery. With a brief reference to Figure 3, it will be appreciated by the skilled artisan that ribbon 26 hinders or prevents the bending of the distal end of guide wire 10 in a direction perpendicular to the direction indicated by arrow 28. Specifically, ribbon 26 is intended to limit or prevent the bending of guide wire 10 in the direction indicated by arrow 30 in Figure 3. Thus, any preset deflection in the guide wire 10 will have a predictable orientation which enhances the controllability of guide wire 10. Both Figure 2 and Figure 3 show that ribbon 26 terminates with a bulb-shaped anchor 32 which provides structure to which the ball-shaped abutment 16 can be securely attached as disclosed above.

Still referring to Figure 2, it can be seen that wire coil 18 surrounds main shaft 14 in the area immediately proximal to abutment 16. Specifically, coil 18 is a helical spring that is made of a radiopaque 88/12 gold alloy similar to that used for abutment 16. As shown in Figure 2, coil 18 has a base portion 34 wherein the wire of coil 18 is tightly wound with a low helical pitch to provide some degree of stiffness. Distal to base portion 34, however, is a flexible portion 36 where the wire of coil 18 is not so tightly wound. As shown, flexible portion 36 surrounds ribbon 26 so that the flexbility of ribbon 26 in the directions indicated by arrow 28 is not impaired. In the contemplation of the present invention, coil 18 is fixedly attached to main shaft 14 and abutment 16. Specifically, coil 18 is soldered to main shaft 14 in the taper region 24 at solder connection 38. The end of coil 18 opposite from connection 38 is brazed onto abutment 16 at braze connection 40 to effectively make coil 18 integral with abutment 16. It is to be appreciated that, rather than being a separate structure which is brazed onto coil 18, abutment 16 can be actually formed from coil 18. In this case, coil 18 is soldered to main shaft 14 as disclosed above and an excess amount of coil 18 which extends beyond ribbon 26 is melted to fuse onto anchor 32 and form abutment 16. For emphasis, and regardless of the method of manufacture, it is again stated that the outer diameter of coil 18 is sufficiently less than the outer diameter of ball-shaped abutment 16 in order to create a barrier at abutment 16 beyond which a device, such as cutter 20, cannot proceed.

It will be appreciated by the skilled artisan that the combination of abutment 16 and coil 18 provides a radiopaque marker which can be used by the physician to properly place guide wire 10 for its intended use. Further, it will be appreciated that the cooperation between ribbon 26 and flexible portion 36 allows the clinician to predictably bend guide wire 10 at a predetermined location in a specifically selected direction to assist in the proper placement of guide wire 10. When once placed, guide wire 10 will be effective for the subsequent placement of over-the-wire catheters and their associated medical devices.

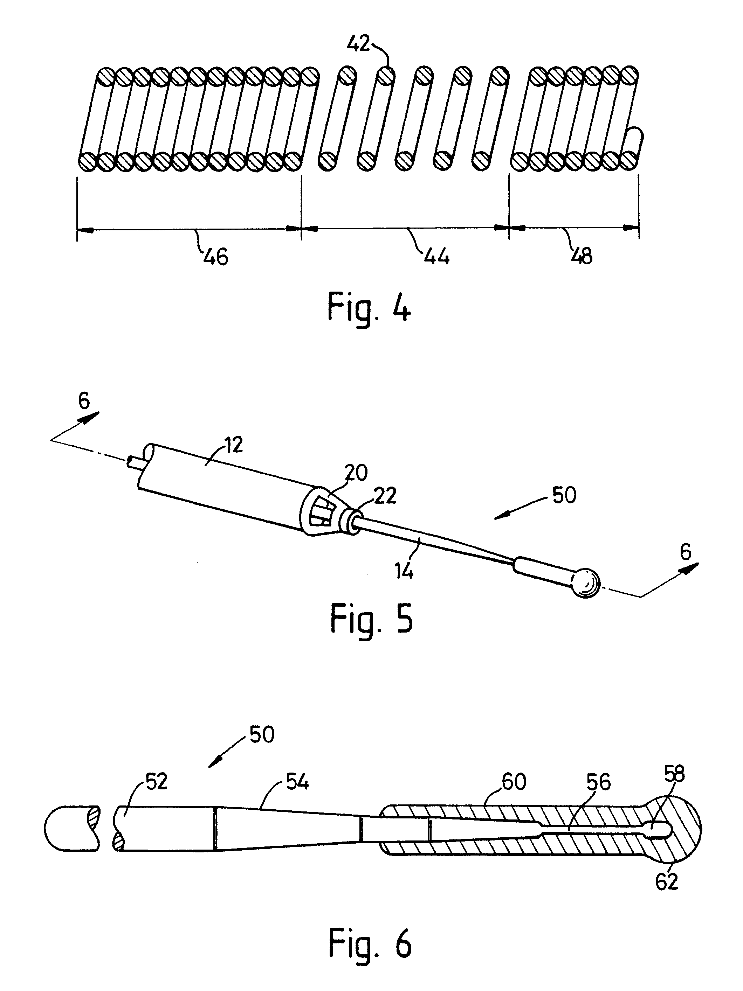

Figure 4 shows a wire coil 42 which can be used as an alternate embodiment of the coil 18. With the coil 42, a more openly wound flexible portion 44 is established between a tightly wound base portion 46 and a tightly wound end portion 48. As with the preferred embodiment of coil 18, the alternate embodiment of coil 42 is preferably made of an 88/12 radiopaque gold alloy and is attached at its distal end to abutment 16 and at its proximal end to a point on tapered region 24 of main shaft 14. With this connection, flexible region 44 surrounds ribbon 26. For all essential purposes, coil 42 functions the same as coil 18 and, like coil 18, is soldered to main shaft 14 and brazed onto abutment 16 to become effectively integral therewith.

An alternate embodiment of a guide wire in accordance with the present invention is shown and generally designated 50 in Figure 5. Before proceeding, however, it is to be appreciated that in its operational effect the alternate embodiment of the present invention functions substantially similar to that of the preferred embodiment. The intended structure of the alternate embodiment can perhaps best be understood by reference to Figure 6 where it will be seen that guide wire 50 comprises a main shaft 52 which is tapered in a region 54 at its distal end. Tapered region 54 is of decreasing diameter in a direction toward the distal end of guide wire 50. Tapered region 54 of guide wire 50 is eventually formed into a flat ribbon 56 which is similar in structure and similar in its intended purpose to the ribbon 26 previously disclosed for guide wire 10 of the preferred embodiment. As also shown in Figure 6, flat ribbon 56 is integrally attached to an anchor 58. Surrounding anchor 58, ribbon 56 and the portion of tapered region 54 immediately proximal to ribbon 56 is a polymer sheath 60. Preferably, polymer sheath 60 is made of a radiopaque material, such as gold filled polyurethane, in order to permit its use as a marker for placement of guide wire 50 in accordance with the desires of the operator. Polymer sheath 60 is formed with a ball portion 62 at the distal end of guide wire 50 which surroundingly engages anchor 58. Sheath 60 and its associated ball portion 62 can be placed over the indicated portions of guide wire 50 in any manner well known in the art, such as by injection molding or casting.

Importantly, ball portion 62 of sheath 60 has a diameter which is sufficiently larger than the outer diameter of main shaft 52 to prevent the movement of over-the-wire catheter 12 past ball portion 62. Thus, in all important respects, the function of ball portion 62 is similar to that of abutment 16 as previously disclosed in the preferred embodiment. Further, in all important respects, guide wire 50 of the alternate embodiment performs substantially the same functions as previously disclosed for guide wire 10 in the preferred embodiment.

## OPERATION

In its operation, guide wire 10 of the present invention is intended to be positioned into the body through a vein or an artery of a patient in accordance with the desires of the operator. Once guide wire 10 is properly placed within the vein or artery, over-the-wire catheter 12 may then be inserted over guide wire 10 and into position along guide wire 10 as desired by the operator. It will be understood that this is done with guide wire 10 inside the lumen of over-the-wire catheter 12. Additionally, it will be understood that over-the-wire catheter 12 can be held stationary while guide wire 10 is further advanced into the vein or artery of the patient. Because ball-shaped abutment 16 has a greater diameter than main shaft 14 of guide wire 10, it is possible to limit the advancement of catheter 12 to that point where catheter 12 makes contact with abutment 16. Specifically, where a cutter 20 with its tip 22 is used, abutment 16 will prevent insertion of cutter 20 past abutment 16. Another advantage of this cooperation of structure is that when guide wire 10 is withdrawn, catheter 12 and its associated medical device, e.g. cutter 20, will be simultaneously withdrawn.

While the particular guide wire as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as defined in the appended claims.

**Claims**

1. A guide wire for positioning an over-the-wire medical device which guide wire comprises:
a main shaft having a proximal end and a distal end; and
an abutment on said distal end of said main shaft to prevent movement of said medical device past said abutment.

2. A guide wire according to Claim 1 wherein said main shaft is formed with a flat ribbon proximal said abutment to bias bending of said main shaft.

3. A guide wire according to Claim 2 further comprising a coil which is positioned in a surrounding relationship over said ribbon, said coil having one end attached to said abutment and an opposite end attached to said main shaft.

4. A guide wire according to Claim 3 wherein the flat ribbon is distal to a tapered region of said main shaft.

5. A guide wire according to Claim 4 wherein said coil is helically wound wire and is surroundingly positioned over at least a portion of said tapered region.

6. A guide wire according to Claim 3, 4 or 5 wherein the portion of said coil in surrounding relationship with said ribbon is more openly wound than is the portion of said coil in surrounding relationship with said tapered region to provide more bending flexibility for said guide wire at said ribbon.

7. A guide wire according to any of Claims 3 to 6 wherein the cross-sectional dimensions of said coil are dimensionally less than the cross-sectional dimensions of said abutment.

8. A guide wire according to any preceding Claim further comprising an anchor integrally attached to said ribbon for providing attachment means for said abutment.

9. A guide wire according to any of Claims 3 to 8 wherein said main shaft is made of stainless steel and said coil and said abutment are made of gold alloy.

10. A guide wire according to Claim 2 wherein said main shaft has a tapered distal end; and said wire comprises a sheath engageable with said distal end, said sheath formed with said abutment for preventing said movement.

11. A guide wire according to Claim 10 further comprising an anchor integrally attached to said distal end of said main shaft and wherein said sheath is formed to surroundingly engage said anchor.

12. A guide wire according to Claim 10 or 11 wherein said sheath is a radiopaque polymer.

13. A guide wire according to any preceding Claim wherein said abutment is ball-shaped.

14. A guide wire according to any of Claims 1 to 12 wherein said abutment is spherical-shaped or hemispherical-shaped, or parabolic-shape

Fig. 1

Fig. 2

Fig. 3

7

Fig. 4

Fig. 5

Fig. 6